# EUROPEAN PATENT APPLICATION

(11) **EP 4 119 944 A1**
(43) Date of publication of application: **18.01.2023**
(21) Application number: 22178896.1
(22) Date of filing: 21.06.2017
(51) Int. Cl.: G01N 33/53

(54) **IMPROVED PHARMACOKINETIC ASSAYS FOR IMMUNOGLOBULIN SINGLE VARIABLE DOMAINS**

(30) Priority: 23.06.2016 US 201662353784 P
(62) Divisional of application: 17737492.3
(71) Applicant: Ablynx N.V., 9052 Ghent-Zwijnaarde (BE)
(72) Inventor: Snoeck, Veerle, 9750 Zingem (BE); Bontinck, Lieselot, 9040 Sint-Amandsberg (BE); Poelmans, Sofie, 9041 Oostakker (BE); Mortier, Kjell, 9810 Eke (BE); Buyse, Marie-Ange, 9820 Merelbeke (BE); Dekeyzer, Lies, 9990 Maldegem (BE); Baumeister, Judith, 2800 Mechelen (BE)
(74) Representative: Hoffmann Eitle

(57) **Abstract**

The present invention generally relates to improved pharmacokinetic assays for measuring levels of immunoglobulin single variable domains (also referred to herein as "ISVs" or "ISVDs") and of proteins and polypeptides that comprise at least one ISV (as further described herein) in biological samples.

## Description

The present invention generally relates to improved pharmacokinetic assays for measuring levels of immunoglobulin single variable domains (also referred to herein as "ISVs" or "ISVDs") and of proteins and polypeptides that comprise at least one ISV (as further described herein) in biological samples.

In particular, the invention relates to improved methods for performing such assays.

Other aspects, embodiments, uses and advantages of the invention will become clear from the further description below.

In drug development, pharmacokinetic (PK) analysis of the drug is performed to determine the fate of the drug (e.g., distribution, absorption, and/or secretion) after administration to an animal or human. Generally, such analysis involves determining the presence, level or concentration of the drug in a biological sample obtained from a subject (like blood, serum or plasma), often at multiple points in time.

For this purpose, pharmacokinetic assays are used, and methods and techniques for performing such assays are generally known to the skilled person. Generally, the usual methods for performing pharmacokinetic assays involve the steps of contacting the sample with a capturing agent that can bind the compound to be measured (i.e. under conditions such that said capturing agent can capture the compound to be measured) and then determining the amount of compound to be measured that has been captured by the capturing agent (which serves as a measure for the amount of compound in the sample). In practice, often a "sandwich" configuration is used, in which the capturing agent is immobilized on a surface (such as the surface of a multi-well plate or chip) and the amount of compound captured by the capturing agent is determined by adding a detection agent that can generate a signal that can be detected and/or measured. In quantitative PK assays, the strength of this signal is a measure of the amount of the compound to be measured that has been captured by the capturing agent, which in turn is a measure for the amount of compound to be measured in the starting sample.

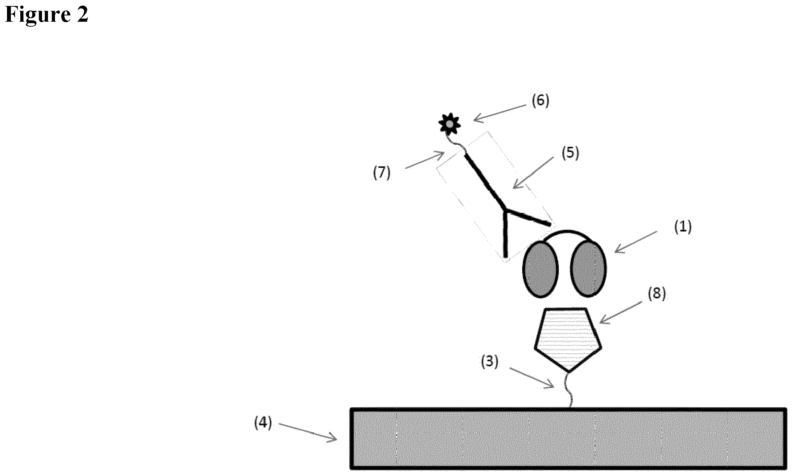

As mentioned, suitable techniques for performing these assays (such as ELISA, the MSD-platform from MesoScale Diagnostic LLC, the Gyrolab^{™} platform from Gyros AB and the Singulex^{™} platform from Merck Millipore) are well-known in the art. Often, the capturing agent is an antibody (polyclonal but preferably monoclonal) that can bind to (and often has been specifically raised against) the compound to be measured (although the present invention also envisages the use of other capturing agents, like ISVDs or the target of the compound to be measured; see for example Figures 2, 4 and 5 and the further description herein). The detection agent can be any suitable agent that can be used to provide a detectable/measurable signal. For example, the detection agent can be a (preferably monoclonal) antibody against the compound to be measured that has been conjugated with a detectable label or tag. Some non-limiting examples that are often used in practice include fluorescent labels, a label or tag that can be detected using electrochemo-luminescence techniques (like the ruthenium-based SULFO-TAG ^{™} labels used as part of the MSD platform), or horseradish peroxidase or another enzyme that can convert a substrate into a detectable/measurable product. As is well known for sandwich immunoassays (like sandwich ELISA), it is also possible to use, as the detection agent, a combination of a detecting antibody that binds to the compound to be measured and an enzyme-linked secondary antibody that can catalyze the conversion of a substrate into a detectable product. For a general description of these techniques and similar techniques, reference is made to the standard handbooks, as well as for example to 'O Kennedy et al., Biochemical Education 18(3), 1990, and Gan and Patel, Journal of Investigative Dermatology (2013), 133, e12. doi:10.1038/jid.2013.287 (online publication).

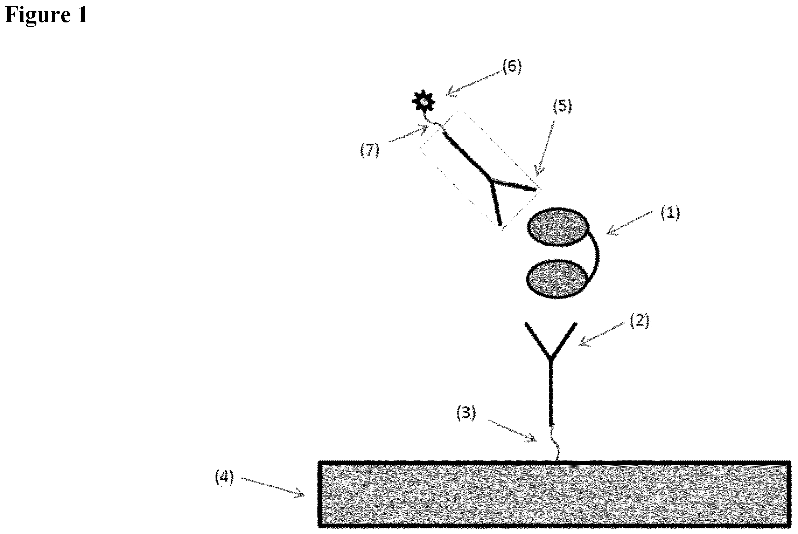

Figures 1 to 6 schematically show some preferred, but non-limiting set-ups for performing pharmacokinetic assays of the kind envisaged by the present application.

In Figure 1 and in the other Figures, the invention is illustrated by using a bivalent ISVD construct (i.e. comprising two ISVD's that are each represented by an oval and that in the illustrated constructs are linked to each other via a suitable linker represented by a solid line) as the compound to be measured (indicated as (1) in each of Figures 1-6). It should be noted that, as further described herein, the choice of this bivalent ISVD construct is for illustration purposes only and that the invention can generally be applied to any assay for determining the amount in a biological sample of any protein, polypeptide or other compound or construct that comprises at least one ISVD (and in particular that, as described herein, comprises at least one ISVD with an exposed C-terminal end, i.e. such that said C-terminal end - and by extension, the protein, polypeptide, compound or construct - is at risk of being bound by pre-existing antibodies in the sample while the assay is being performed). Other examples of such proteins, polypeptides, compounds or constructs will be clear to the skilled person based on the disclosure herein, and as mentioned herein for example include monovalent, bivalent, trivalent, monospecific, bispecific, trispecific and/or biparatopic ISVD polypeptides or constructs (for example, in Example 1, ALX-0171, a trivalent Nanobody construct against RSV is used).

In the assay of Figure 1, a monoclonal antibody (2), linked to a solid support (4) via a covalent bond or other suitable linker or immobilization technique (3) is used to capture the compound to be measured (1). After removing any unbound compound (1) (i.e. by washing), the amount of compound (1) captured by monoclonal (2) is determined by adding the detection agent, which in the set-up shown in Figure 1 is a monoclonal antibody (5) conjugated with a detectable label (6) via a linker (7) or another suitable conjugation technique (such as a streptavidin-biotin pair). Any excess detection agent is then removed (i.e. again by washing), after which the amount of detection agent bound to the compound (1) captured by the capturing agent is determined, using the signal provided by the detectable label (6).

The assay set-up shown in Figure 2 is essentially the same as the set-up shown in Figure 1, except that, instead of a monoclonal antibody against the compound (1), the target (8) of the compound (1) is immobilized on the solid support (4) (i.e. using the linker (3).

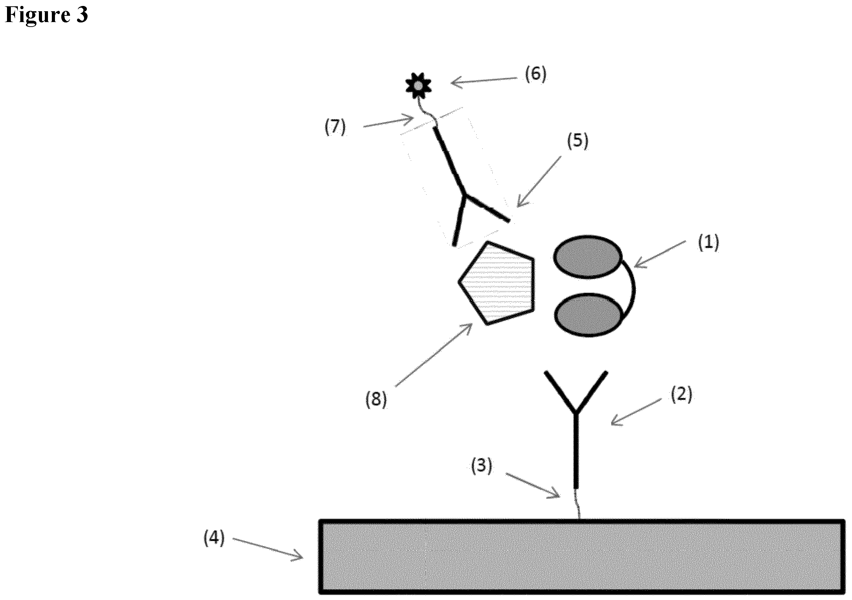

In the assay set-up shown in Figure 3, the compound to be measured (1) is again captured by the monoclonal antibody (2) linked to the solid support (4), but in this case the assay is performed in the presence of the (soluble) target (8) of the Nanobody construct (1) to be measured, and the detection agent (comprising the monoclonal (5) and the detectable label (6), linked by the linker (7)) is directed towards the target (8) rather than the compound (1).

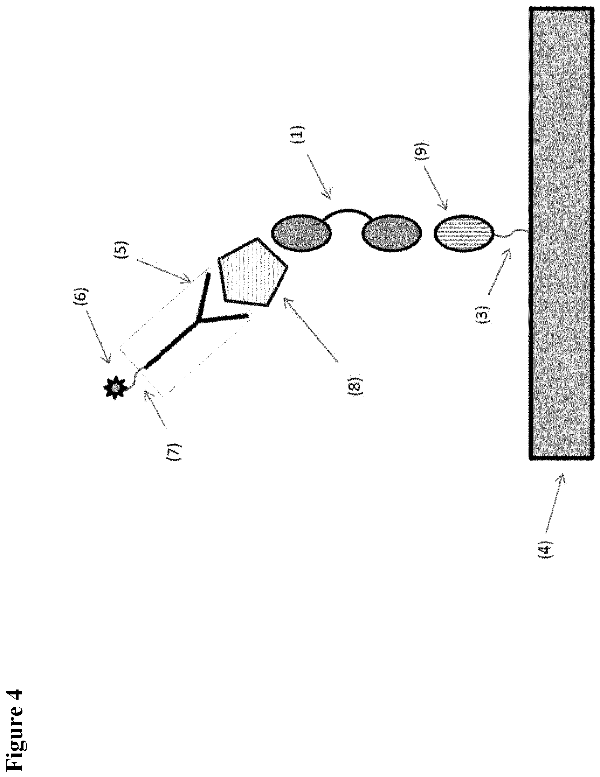

The assay set-up shown in Figure 4 is essentially the same as the set-up shown in Figure 3, except that an "anti-ISVD ISVD" (9) (e.g. an Nanobody directed against the framework sequences of VHHs) is used as the capturing agent instead of a monoclonal antibody.

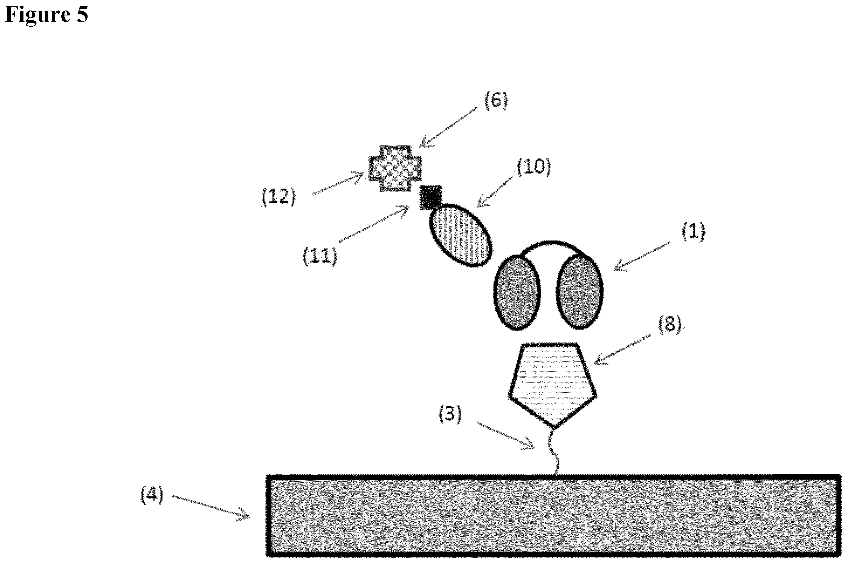

The assay set-up shown in Figure 5 is essentially the same as the set-up shown in Figure 2, except that, instead of the monoclonal antibody (5), a combination of a biotinylated (11) anti-ISVD ISVD (10) and horseradish peroxidase (HRP) labelled streptavidin (12) is used as the detection agent.

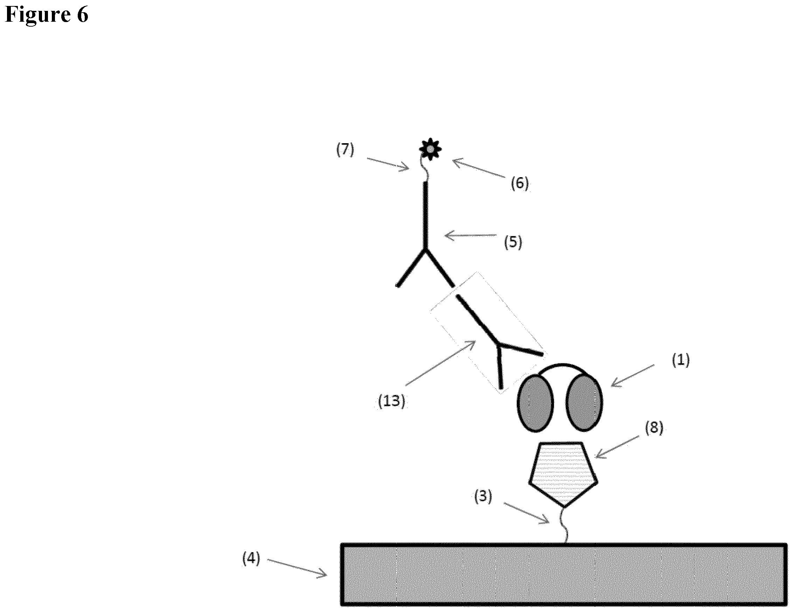

The assay set-up shown in Figure 6 is essentially the same as the set-up shown in Figure 2, except that as the detection agent, a combination of a detection antibody (13) and a labelled secondary antibody (5)/(6)/(7) is used.

Suitable assay set-ups other than those explicitly described in the Figures will be clear to the skilled person based on the disclosure and examples herein, and as mentioned, the assays shown in Figures 1-6 and similar assays can be performed using generally known techniques and methodology which will be clear to the skilled person based on the disclosure herein.

In WO 12/175741, it is described that protein interference may occur in some assays (such as, for example, in ADA immunoassays) that are used for analyzing biological samples (such as blood samples including whole blood, serum and plasma, ocular fluid, bronchoalveolar fluid/BALF, cerebrospinal fluid or other samples of biological fluids) containing ISVD's, and that such protein interference may give rise to an aspecific signal in some of these assays and/or for some of these samples. WO 12/175741 also mentions that such protein interference may occur not only in samples that are obtained from subjects that have been treated with an ISVD and/or to whom an ISVD has been administered (such as patients or clinical trial subjects), but also in samples from subjects that have never received an ISVD. As further mentioned in WO 12/175741, this indicates that such interference is likely due to an aspecific protein-protein interaction with pre-existing proteins rather than with any emerging ADA's. WO 12/175741 further indicates that these interfering factors are most likely (pre-existing) IgG's that can bind to the C-terminal end of a variable domain, if it is exposed (see for example also WO 13/024069, Holland et al., J. Clin. Immunol. 2013, 33(7): 1192-203 and WO 2015/173325).

As pharmacokinetic assays generally involve the use of serum samples (i.e. from subjects to which an ISVD or a drug comprising an ISVD has been administered), it is possible that such "pre-existing antibodies" against an exposed C-terminal end of an ISVD, when they are present in such samples, may interfere with said assay. In view of this, the invention aims to provide improved pharmacokinetic assays and methods for performing the same in which such interference (and/or the risk of such interference occurring) is reduced.

Generally, the invention solves the problem of possible protein interference by performing the assay in the presence of (a sufficient amount of) a "quencher", i.e. a protein or polypeptide to which any pre-existing antibodies that are present in the sample can bind. As a result, the interfering factors are not (or no longer) capable of interfering with the association between the capturing agent, the compound to be measured and the detection agent (i.e. in a manner that could affect or distort the assay).

Generally, as further described herein, the quenchers used herein are ISVD's or proteins and polypeptides that comprise an ISVD with an exposed C-terminal end, which ISVDs, protein or polypeptides are further such that they cannot be captured by the capturing agent and are not bound by the compound to be measured or the detection agent.

Thus, in a first aspect, the invention relates to a method for determining the amount and/or concentration in a sample of at least one ISVD, or of a protein, polypeptide or other compound or construct that comprises at least one ISVD (which protein, polypeptide or other compound or construct is as described herein), which method comprises the steps of;
a) contacting the sample with a capturing agent, such that the ISVD, protein, polypeptide, compound or construct is captured by said capturing agent;
b) contacting the ISVD, protein, polypeptide, compound or construct captured by the capturing agent with a detection agent, such that said detection agent binds to the ISVD, protein, polypeptide, compound or construct captured by the capturing agent;
c) generating a signal corresponding to the amount of detection agent bound to the ISVD, protein, polypeptide compound or construct that has been captured by the capturing agent, in which said method is performed in the presence of a quencher, said quencher being a protein or polypeptide to which pre-existing antibodies can bind (i.e. specifically bind) but to which the capturing agent and the detection agent (essentially) cannot bind (i.e. other than aspecific binding). For this purpose, the quencher preferably is as further described herein.

As will be clear to the skilled person based on the disclosure herein, generally, in the method described above, the sample will be a sample of a biological fluid that is known to contain and/or suspected to contain pre-existing antibodies that are directed against the exposed C-terminal end of an ISVD. In particular, the sample will be a sample of a biological fluid that is known to contain and/or suspected to contain pre-existing antibodies that are directed against the exposed C-terminal end of an ISVD (and in particular against a Nanobody or another ISVD that is or has been derived from a VH or VHH domain).

The sample will further contain the (at least one) ISVD, protein, polypeptide, compound or construct to be measured. In particular, said ISVD, protein, polypeptide, compound or construct to be measured may be an ISVD, protein, polypeptide, compound or construct that has been administered to the subject from whom the sample has been obtained, i.e. as part of a clinical trial or for therapeutic or diagnostic purposes. In one aspect, the sample has been obtained in order to determine the pharmacological properties of the ISVD, protein, polypeptide, compound or construct to be measured, and in particular its pharmacokinetic properties (e.g. its PK or serum half-life parameters).

The sample may be any desired and suitable sample of a biological fluid obtained from a subject, such as a sample of whole blood, serum, plasma, lymph fluid, ocular fluid, bronchoalveolar fluid/BALF, cerebrospinal fluid or another biological fluid (such as sputum or nasal washes); and in particular a sample of whole blood, serum or plasma. Said sample may also be/have been suitably prepared for use in the assay of the invention (for example, by suitable dilution or extraction methods if appropriate, and/or by one or more suitable pretreatments steps known per se in the art, such as a suitable acid dissociation step). In practice, usually, the sample will be known to contain, or expected to contain, a certain amount of the ISVD, protein, polypeptide compound or construct to be measured, for example because - as mentioned herein - said sample has been obtained from a subject to which said ISVD, protein, polypeptide compound or construct has been administered.

The ISVD, protein, polypeptide compound or construct to be measured using the assay of the invention may comprise or essentially consist of a monovalent ISVD or may be a protein, polypeptide, compound or construct that comprises or essentially consists of one or more (such as one, two three, four or five) ISVDs. These may for example include proteins, polypeptides, compounds or constructs that comprise at least one (such as one, two or three) ISVDs, and optionally one or more other moieties or entities, suitably linked to each other, for example via direct chemical linkage or using one or more suitable linkers. Reference is made to the further description and prior art referred to herein.

In one aspect, in such proteins, polypeptides, compounds or constructs, at least one of the ISVD's present will be directed against a therapeutically relevant target. In one specific aspect, such proteins, polypeptides, compounds or constructs will have a half-life in man (expressed as t1/2-beta) of at least one day, such as at least three days, for example up to 5 days or more. For this purpose, the protein, polypeptide, compound or construct may contain a moiety, binding domain or binding unit that provides the at least one "therapeutic" ISVD with such increased half-life. This may for example be an ISVD against a (human) serum protein such as against (human) serum albumin. Reference is made to the further disclosure herein.

In another aspect, such proteins, polypeptides, compounds or constructs will comprise at least one an ISVD against a (human) serum protein such as (human) serum albumin, for which again reference is made to the further disclosure herein. Generally, in this aspect, the protein, polypeptide, compound or construct will usually further contain at least one therapeutically active moiety, binding domain or binding unit, such as one or more binding domains or binding units (again including ISVDs such as Nanobodies) against one or more therapeutically relevant target(s). In such proteins, polypeptides, compounds or constructs, the serum albumin-binding ISVD will generally be used to increase the half-life of the one or more therapeutic moieties or entities. Again, such proteins, polypeptides, compounds or constructs will have a half-life in man (expressed as t1/2-beta) of at least one day, such as at least three days, for example up to 5 days or more.

The proteins and polypeptides referred to herein are preferably fusion proteins. In one aspect, the proteins and polypeptides comprise or essentially consist of ISVD's, optionally linked via one or more suitable linkers.

Generally, in the invention, the ISVD, protein, polypeptide, compound or construct to be measured will be such that it comprises at least one ISVD (such as an ISVD against a therapeutic target and/or a half-life extending ISVD such as an ISVD against a serum protein) that is susceptible to, and/or at risk of, being bound by pre-existing antibodies, and in particular by pre-existing antibodies against the exposed C-terminal end of said ISVD. Often, said ISVD will be at the C-terminal end of the protein, polypeptide, compound or construct to be measured.

In one particular aspect, the ISVD, protein, polypeptide, compound or construct to be measured will be such that it comprises at least one heavy-chain ISVD (i.e. an ISVD that is a Nanobody or another ISVD that is or has been derived from a VHH or VH domain, as further described herein) that is susceptible to, and/or at risk of, being bound by pre-existing antibodies, and in particular by pre-existing antibodies against the exposed C-terminal end of said ISVD. Often, said heavy-chain ISVD will be at the C-terminal end of the protein, polypeptide, compound or construct to be measured.

The one or more ISVDs present in the ISVD, protein, polypeptide, compound or construct to be measured may also be sequence-optimized for reduced binding by pre-existing antibodies, for example by having a C-terminal extension (as for example described in WO 12/175741) and/or by having framework mutations that reduce binding by pre-existing antibodies (see WO 2015/173325).

For the ISVDs, proteins, polypeptides, compounds or constructs that can be measured using the methods and assays of the invention, further reference is made to the disclosure and prior art cited herein.

The quencher used may be any ISVD that has an exposed C-terminal end, or any protein, polypeptide, compound or construct that comprises at least one ISVD with an exposed C-terminal end (and in particular, with an ISVD at its C-terminal end). For example, in one specific, but non-limiting aspect, the quencher comprises two (or may even comprise three) ISVD's linked to each other using a suitable linker known per se.

The quencher will be chosen such that any pre-existing antibodies in the sample can (specifically) bind to it, in particular with an affinity that is as described herein.

To ensure that the pre-existing antibodies will bind to the quencher (i.e. rather than to the protein, polypeptide, compound or construct to be measured), the quencher may be chosen such that the affinity for the binding interaction between the quencher and the pre-existing antibodies is higher/better (as described herein) than the affinity for the binding interaction between the protein, polypeptide, compound or construct to be measured and the pre-existing antibodies (for example and without limitation, when the protein, polypeptide, compound or construct to be measured contain a C-terminal extension and/or framework mutations that reduce binding by pre-existing antibodies, this can easily be achieved by chosen a quencher that does not contain such C-terminal extension and such mutations).

Thus, in one non-limiting aspect, the quencher used in the methods of the invention is chosen such that affinity for the binding interaction between the quencher and the pre-existing antibodies is at least 10 times (such as at least 100 times) higher/better (as described herein) than the affinity for the binding interaction between the protein, polypeptide, compound or construct to be measured and the pre-existing antibodies (in (generally, in the context of the present application, by means of illustration, an affinity of 10nM is considered 10x "better" or "higher" than an affinity of 100nM).

As further described herein, for the purposes of comparing affinities as described in the previous paragraphs, the affinity for the binding interaction between the quencher and the antibody 21-4 (which can be used as a model/surrogate for pre-existing antibodies) can be compared to the affinity for the binding interaction between the protein, polypeptide, compound or construct to be measured and the antibody 21-4. Thus, in one specific but non-limiting aspect, in the methods of the invention, the quencher may be chosen such that the affinity for the binding interaction between the quencher and the antibody 21-4 is better/higher (as defined herein, and in particular at least 10 times better such as at least 100 times better) than the affinity for the binding interaction between the protein, polypeptide, compound or construct to be measured and the antibody 21-4.

Another way of ensuring that the pre-existing antibodies will bind to the quencher (i.e. rather than to the protein, polypeptide, compound or construct to be measured) is to use a suitable excess (such as at least 10 times excess, for example at least 100 times excess) compared to the amount of protein, polypeptide, compound or construct to be measured that is (maximally) expected to be present in the sample. Generally, such a suitable excess should be used when (it is expected that) the affinity for the binding interaction between the quencher and the pre-existing antibodies is about the same or even worse/lower than the affinity for the binding interaction between the protein, polypeptide, compound or construct to be measured and the pre-existing antibodies (generally, in the context of the present application, by means of illustration, an affinity of 100nM is considered 10x "worse" or "lower" than an affinity of 10nM). Also, a suitable excess of quencher can also be used when (it is expected that) the affinity for the binding interaction between the quencher and the pre-existing antibodies is better than the affinity for the binding interaction between the protein, polypeptide, compound or construct to be measured and the pre-existing antibodies.

Preferably, the quencher (and in particular the ISVD in the quencher to which the pre-existing antibodies are intended to bind) has no sequence optimization to reduce binding by pre-existing antibodies. For example, it may end with the sequence VTVSS without any C-terminal extension, and it preferably also does not contain mutations that are intended to reduce binding by pre-existing antibodies. Apart from that, and ISVD or protein, polypeptide, compound or construct comprising an ISVD with an exposed C-terminal end may be used, as long as it does not interfere with the assay (for example, because it can bind to, and/or is bound by, any of the other components used in the assay).

In practice, with regard to (possible) binding of the quencher by pre-existing antibodies and also with regard to selection of a suitable quencher to which pre-existing antibodies can bind, it is remarked that it is possible that the pre-existing antibodies in a sample may form a heterogeneous polyclonal population and that it is also possible that pre-existing antibodies (and their affinities for ISVDs) may sometimes also differ from sample to sample and/or from subject to subject.

To account for this, in the invention, binding (i.e. affinity) by the mouse monoclonal antibody clone "21-4-3" (also referred to herein as "21-4" or "ABH0015") can be used as a model/tool to determine whether a candidate quencher will be bound by pre-existing antibodies or not. 21-4-3 (and the hybridoma producing it, also called "ABH0015") is known from WO 12/175741 (see page 19, lines 3-28) where it is described and used as a surrogate/model for determining whether a protein or polypeptide that contains an ISVD with an exposed C-terminal end will have a tendency to undergo protein interference (i.e. be bound by pre-existing antibodies that may be present in a biological sample that has been obtained from a subject). WO 12/175741 also gives the VH and VL sequences of ABH0015 (see WO 12/175741, SEQ ID NOs: 35 and 36). As mentioned in Example 7 of WO 12/175741, 21-4 was generated using hybridoma technology starting from a mouse immunized with the Nanobody construct of SEQ ID NO:98 in WO 2006/122825, and a hybridoma cell line (called "ABH0015") expressing 21-4 has been deposited on June 4, 2012 with the BCCM, Ghent, Belgium, under accession number LMBP-9680-CB. As shown in Example 8 of WO 12/175741, monoclonal 21-4 recognizes the C-terminus of the Nanobody construct of SEQ ID NO:98 in WO 2006/122825, which C-terminal end consists of a Nanobody (humanized VHH) raised against Von Willebrand Factor (vWF), and can be used in order to predict whether an ISV has a tendency to undergo aspecific protein interference. In Example 9, WO 12/175741 also gives a protocol in which ABH0015 is used to predict/determine whether a protein or polypeptide (in particular, a protein or polypeptide comprising an ISVD with an exposed C-terminal end) will or will not have a tendency to undergo protein interference (i.e. be bound by pre-existing antibodies that may be present in a biological sample obtained from a subject).

In the present invention, binding to/by 21-4 can be used as a surrogate to measure/ determine whether a protein or polypeptide will be suitable for use as a quencher or not (see also Example 5 herein).

Generally, in the invention, a protein or polypeptide will be suitable for use as a quencher if it is bound by 21-4 with an affinity (expressed as KD) better than 1 micromolar (µM), such as between 1000 and 1 nM, when determined using BIAcore according to the protocol described in Example 5 (for the sake of clarity, and by means of non-limiting example, it is remarked that generally, in the present description of the invention, an affinity of 1 nM is considered to be "higher/better" than an affinity of 1 micromolar/1000nM).

As mentioned, when in the methods of the invention it is intended to use a quencher that has higher/better affinity for pre-existing antibodies than the affinity which with the ISVD, protein, polypeptide, compound or construct to be measured binds to pre-existing antibodies, then preferably, the quencher used will bind to 21-4 with an affinity that is a factor 10x (ten times) higher/better, preferably a factor 100x (100 times) times higher/better, than the affinity which with the ISVD, protein, polypeptide, compound or construct to be measured binds to 21-4 (again, in this context, by means of illustration, an affinity of 10nM is considered to be 10 times "higher" than an affinity of 100nM).

Thus, in one aspect, the invention relates to a method as described hereinabove, in which the protein or polypeptide used as the quencher is a protein or polypeptide that is bound by 21-4 with an affinity better than 1 micromolar (µM), such as between 1000 and 1 nM, when determined using BIAcore according to the protocol described in Example 5.

The quencher should also essentially not be bound by (or not bind to, other than aspecific binding) the capturing agent or the detection agent. In practice, this means that the affinity of the quencher for the capturing agent and for the detection agent (or vice versa, the affinity of the capturing agent for the quencher and the affinity of the detection agent for the quencher) should be less/worse than 3 micromolar, preferably less than 10 micromolar, more preferably less than 50 micromolar, such as worse than 100 micromolar (for the sake of clarity, and by means of non-limiting example, it is remarked that generally, in the present description of the invention, an affinity of 10 micromolar is considered to be "worse/less" than an affinity of 1 micromolar). Once a (candidate) capturing agent and a (candidate) detection agent have been selected, it should be well within the skill of the artisan to determine the affinity of the same for a (candidate) quencher and so select a quencher that is suitable for use with the intended capturing agent and detection agent.

Thus, in a further aspect, the invention relates to a method as described hereinabove, in which the protein or polypeptide used as the quencher is a protein or polypeptide that: (i) is bound by 21-4 with an affinity better than 1 micromolar (µM), such as between 1000 and 1 nM, when determined using BIAcore according to the protocol described in Example 5; and (ii) is bound by the intended capturing agent and the intended detection agent with an affinity less/worse than 3 micromolar, preferably less than 10 micromolar, more preferably less than 50 micromolar, such as worse than 100 micromolar.

In the invention, the quencher used can be a monovalent ISVD (such as a monovalent Nanobody) or a fusion protein or construct comprising two or more (such as two or three) ISVDs (of which at least one has an exposed C-terminal end). In practice, the quencher will usually be a polypeptide, (fusion) protein or construct that has an ISVD at its C-terminal end (which ISVD will then have an exposed C-terminal end by virtue of forming the C-terminal end of the polypeptide, protein or construct).

As the quencher should be able to be bound by pre-existing antibodies, the VHH that forms the C-terminal end of the quencher preferably will not have any C-terminal extension (as described in WO 12/175741) nor any mutations that are intended to reduce binding by pre-existing antibodies (like those described in WO 2015/173325).

Accordingly, and preferably, the quencher is a protein or polypeptide that has an ISVD (and preferably a Nanobody) at its C-terminal end, and (the ISVD that forms the C-terminal end of) the quencher preferably has the amino acid sequence VTVSS (SEQ ID NO: 1) at its C-terminal end. The quencher may for example suitably be a monovalent, bivalent, bispecific , trivalent or trispecific construct ISVD construct. Preferably, the quencher is a monovalent Nanobody, a bivalent Nanobody construct (which may be monospecific or bispecific) or a trivalent Nanobody construct (which may be monospecific, bispecific or trispecific).

As shown in Figures 2-6, in some possible set-ups for the assay of the invention, the target for the compound to be measured (indicated as (8) in Figures 2 to 6) may be used, either as part of the capturing agent or as part of the detection agent (or both). Accordingly, although the ISVD(s) present in the quencher may be directed against any target(s), it should not be directed against the target of the compound to be measured if said target is used as part of the assay.

Thus, in a further aspect, the invention relates to a method as described hereinabove, in which the protein or polypeptide used as the quencher is a protein or polypeptide that contains at least one ISVD having an exposed C-terminal end, in which the C-terminal end of said ISVD ends with the C-terminal amino acid sequence VTVSS (SEQ ID NO: 1). Again, said quencher preferably: (i) is bound by 21-4 with an affinity better than 1 micromolar (µM), such as between 1000 and 1 nM, when determined using BIAcore according to the protocol described in Example 5; and (ii) is bound by the intended capturing agent and the intended detection agent with an affinity less/worse than 3 micromolar, preferably less than 10 micromolar, more preferably less than 50 micromolar, such as worse than 100 micromolar.

In another aspect, the invention relates to a method as described hereinabove, in which the protein or polypeptide used as the quencher is a protein or polypeptide that has an ISVD at its C-terminal end, in which said C-terminal ISVD (and as a consequence, the protein or polypeptide used as the quencher) ends with the C-terminal amino acid sequence VTVSS (SEQ ID NO:1). Again, said quencher preferably: (i) is bound by 21-4 with an affinity better than 1 micromolar (µM), such as between 1000 and 1 nM, when determined using BIAcore according to the protocol described in Example 5; and (ii) is bound by the intended capturing agent and the intended detection agent with an affinity less/worse than 3 micromolar, preferably less than 10 micromolar, more preferably less than 50 micromolar, such as worse than 100 micromolar.

One convenient way of using the quencher in the methods of the invention is to dilute the samples to be tested (which as mentioned above may already have been subjected to one or more suitable pretreatment steps known per se, such as an acid dissociation step) with a suitable dilution buffer containing (an excess of) the quencher prior to the capturing step. In this way, the pre-existing antibodies in the sample will have been bound by the quencher prior to the capturing and detection steps, thus ensuring that they cannot interfere with the measurement and/or read-out of the assay.

Other aspects, embodiments, advantages and applications of the invention will become clear from the further description herein.

In the present specification:
- the term "immunoglobulin single variable domain" (also referred to as "ISV" or ISVD") is generally used to refer to immunoglobulin variable domains (which may be heavy chain or light chain domains, including VH, VHH or VL domains) that can form a functional antigen binding site without interaction with another variable domain (e.g. without a VH/VL interaction as is required between the VH and VL domains of conventional 4-chain monoclonal antibody). Examples of ISVDs will be clear to the skilled person and for example include Nanobodies (including a VHH, a humanized VHH and/or a camelized VHs such as camelized human VH's), IgNAR, domains, (single domain) antibodies (such as dAb's^{™}) that are VH domains or that are derived from a VH domain and (single domain) antibodies (such as dAb's^{™}) that are VL domains or that are derived from a VL domain. Unless explicitly mentioned otherwise herein, ISVDs that are based on and/or derived from heavy chain variable domains (such as VH or VHH domains) are generally preferred. Most preferably, unless explicitly indicated otherwise herein, an ISVD will be a Nanobody.
- the term "Nanobody" is generally as defined in WO 2008/020079 or WO 2009/138519, and thus in a specific aspect generally denotes a VHH, a humanized VHH or a camelized VH (such as a camelized human VH) or generally a sequence optimized VHH (such as e.g. optimized for chemical stability and/or solubility, maximum overlap with known human framework regions and maximum expression). It is noted that the terms Nanobody or Nanobodies are registered trademarks of Ablynx N.V. and thus may also be referred to as Nanobody^{®} and/or Nanobodies^{®});
- Generally, unless indicated otherwise herein, the ISVD's, Nanobodies, polypeptides, proteins and other compounds and constructs referred to herein will be intended for use in prophylaxis or treatment of diseases or disorders in man (and/or optionally also in warmblooded animals and in particular mammals). Thus, generally, the ISVD's, Nanobodies, polypeptides, proteins and other compounds and constructs described herein are preferably such that they can be used as, and/or can suitably be a part of, a (biological) drug or other pharmaceutically or therapeutically active compound and/or of a pharmaceutical product or composition. Such a drug, compound or product is preferably such that it is suitable for administration to a human being, e.g. for prophylaxis or treatment of a subject in need of such prophylaxis or treatment or for example as part of a clinical trial. As further described herein, for this purpose, such a drug or compound may contain other moieties, entities or binding units besides the ISVDs provided by the invention (which, as also described herein, may for example be one or more other further therapeutic moieties and/or one or more other moieties that influence the pharmacokinetic or pharmacodynamic properties of the ISVD-based or Nanobody-based biological, such as its half-life). Suitable examples of such further therapeutic or other moieties will be clear to the skilled person, and for example generally can include any therapeutically active protein, polypeptide or other binding domain or binding unit, as well as for example modifications such as those described on pages 149 to 152 of WO 2009/138159. An ISVD-based biological or Nanobody-based biological is preferably a therapeutic or intended for use as a therapeutic (which includes prophylaxis and diagnosis) and for this purpose preferably contains at least one ISVD against a therapeutically relevant target (such as for example RANK-L, vWF, IgE, RSV, CXCR4, IL-23 or other interleukins or their receptors, etc.). For some specific but non-limiting examples of such ISVD-based or Nanobody-based biologicals, reference is to Examples 8 to 18 and also for example made to the various applications by Ablynx N.V. (such as for example and without limitation WO 2004/062551, WO 2006/122825, WO 2008/020079 and WO 2009/068627), as well as for example (and without limitation) to applications such as WO 2006/038027, WO 2006/059108, WO 2007/063308, WO 2007/063311, WO 2007/066016 and WO 2007/085814. Also, as further described herein, the further moiety may be an ISVD or Nanobody as described herein directed against a (human) serum protein such as (human) serum albumin, and such an ISVD or Nanobody may also find therapeutic uses, in particular in and/or for extending the half-life of one or more therapeutic ISVDs. Reference is for example made to WO 2004/041865, WO 2006/122787 and WO 2012/175400, which generally describe the use of serum-albumin binding Nanobodies for half-life extension. Also, in the present specification, unless explicitly mentioned otherwise herein, all terms mentioned herein have the meaning given in WO 2009/138519 (or in the prior art cited in WO 2009/138519) or WO 2008/020079 (or in the prior art cited in WO 2008/020079). Also, where a method or technique is not specifically described herein, it can be performed as described in WO 2009/138519 (or in the prior art cited in WO 2009/138519) or WO 2008/020079 (or in the prior art cited in WO 2008/020079). Also, as described herein, any pharmaceutical product or composition comprising any ISVD or compound of the invention may also comprise one or more further components known per se for use in pharmaceutical products or compositions (i.e. depending on the intended pharmaceutical form) and/or for example one or more other compounds or active principles intended for therapeutic use (i.e. to provide a combination product).

Also, when used in the present specification or claims, the following terms have the same meaning as given on, and/or where applicable can be determined in the manner described in, pages 62-75 of WO 2009/138519: *"agonist", "antagonist", "inverse agonist", "non-polar, uncharged amino acid residue", "polar uncharged amino acid residue", "polar, charged amino acid residue", "sequence identity", "exactly the same"* and *"amino acid difference"* (when referring to a sequence comparison of two amino acid sequences), *"(in) essentially isolated (form)* ", *"domain", "binding domain", "antigenic determinant", "epitope", "against"* or *"directed against"* (an *antigen),"specificity"* and *"half-life".* In addition, the terms *"modulating"* and *"to modulate", "interaction site", "specific for", "cross-block", "cross-blocked'* and *"cross-blocking"* and *"essentially independent of the pH"* are as defined on (and/or can be determined as described on) pages 74-79 of WO 2010/130832 of Ablynx N.V.. Also, when referring to a construct, compound, protein or polypeptide of the invention, terms like *"monovalent", "bivalent'* (or *"multivalenf'), "bispecific"* (or *"multispecific"),* and *"biparatopic"* (or *"multiparatopic")* may have the meaning given in WO 2009/138519, WO 2010/130832 or WO 2008/020079.

The term "half-life" as used here in relation to an ISVD, Nanobody, ISVD-based biological, Nanobody-based biological or any other amino acid sequence, compound or polypeptide referred to herein can generally be defined as described in paragraph o) on page 57 of WO 2008/020079 and as mentioned therein refers to the time taken for the serum concentration of the amino acid sequence, compound or polypeptide to be reduced by 50%, in vivo, for example due to degradation of the sequence or compound and/or clearance or sequestration of the sequence or compound by natural mechanisms. The in vivo half-life of an amino acid sequence, compound or polypeptide of the invention can be determined in any manner known per se, such as by pharmacokinetic analysis. Suitable techniques will be clear to the person skilled in the art, and may for example generally be as described in paragraph o) on page 57 of WO 2008/020079. As also mentioned in paragraph o) on page 57 of WO 2008/020079, the half-life can be expressed using parameters such as the t1/2-alpha, t1/2-beta and the area under the curve (AUC). In this respect it should be noted that the term "half-life" as used herein in particular refers to the t1/2-beta or terminal half-life (in which the t1/2-alpha and/or the AUC or both may be kept out of considerations). Reference is for example made to the Experimental Part below, as well as to the standard handbooks, such as Kenneth, A et al: Chemical Stability of Pharmaceuticals: A Handbook for Pharmacists and Peters et al, Pharmacokinetic analysis: A Practical Approach (1996). Reference is also made to "Pharmacokinetics", M Gibaldi & D Perron, published by Marcel Dekker, 2nd Rev. edition (1982). Similarly, the terms "increase in half-life" or "increased half-life" are also as defined in paragraph o) on page 57 of WO 2008/020079 and in particular refer to an increase in the t1/2-beta, either with or without an increase in the t1/2-alpha and/or the AUC or both.

When a term is not specifically defined herein, it has its usual meaning in the art, which will be clear to the skilled person. Reference is for example made to the standard handbooks, such as Sambrook et al, "Molecular Cloning: A Laboratory Manual" (2nd.Ed.), Vols. 1-3, Cold Spring Harbor Laboratory Press (1989); F. Ausubel et al, eds., "Current protocols in molecular biology", Green Publishing and Wiley Interscience, New York (1987); Lewin, "Genes II", John Wiley & Sons, New York, N.Y., (1985); Old et al., "Principles of Gene Manipulation: An Introduction to Genetic Engineering", 2nd edition, University of California Press, Berkeley, CA (1981); Roitt et al., "Immunology" (6th. Ed.), Mosby/Elsevier, Edinburgh (2001); Roitt et al., Roitt's Essential Immunology, 10th Ed. Blackwell Publishing, UK (2001); and Janeway et al., "Immunobiology" (6th Ed.), Garland Science Publishing/Churchill Livingstone, New York (2005), as well as to the general background art cited herein.

Also, as already indicated herein, the amino acid residues of a Nanobody are numbered according to the general numbering for VHs given by Kabat et al. ("Sequence of proteins of immunological interest", US Public Health Services, NIH Bethesda, MD, Publication No. 91), as applied to VHH domains from Camelids in the article of Riechmann and Muyldermans, J. Immunol. Methods 2000 Jun 23; 240 (1-2): 185-195; or referred to herein. According to this numbering, FR1 of a Nanobody comprises the amino acid residues at positions 1-30, CDR1 of a Nanobody comprises the amino acid residues at positions 31-35, FR2 of a Nanobody comprises the amino acids at positions 36-49, CDR2 of a Nanobody comprises the amino acid residues at positions 50-65, FR3 of a Nanobody comprises the amino acid residues at positions 66-94, CDR3 of a Nanobody comprises the amino acid residues at positions 95-102, and FR4 of a Nanobody comprises the amino acid residues at positions 103-113. [In this respect, it should be noted that - as is well known in the art for VH domains and for VHH domains - the total number of amino acid residues in each of the CDR's may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence. Generally, however, it can be said that, according to the numbering of Kabat and irrespective of the number of amino acid residues in the CDR's, position 1 according to the Kabat numbering corresponds to the start of FR1 and vice versa, position 36 according to the Kabat numbering corresponds to the start of FR2 and vice versa, position 66 according to the Kabat numbering corresponds to the start of FR3 and vice versa, and position 103 according to the Kabat numbering corresponds to the start of FR4 and vice versa.].

Alternative methods for numbering the amino acid residues of VH domains, which methods can also be applied in an analogous manner to VHH domains from Camelids and to Nanobodies, are the method described by Chothia et al. (Nature 342, 877-883 (1989)), the so-called "AbM definition" and the so-called "contact definition". However, in the present description, aspects and figures, the numbering according to Kabat as applied to VHH domains by Riechmann and Muyldermans will be followed, unless indicated otherwise.

It should also be noted that the Figures, any Sequence Listing and the Experimental Part/Examples are only given to further illustrate the invention and should not be interpreted or construed as limiting the scope of the invention and/or of the appended claims in any way, unless explicitly indicated otherwise herein.

The invention will now be further described by means of the following non-limiting preferred aspects, examples and figures, in which Figures 1 to 6 schematically illustrate different set-ups for performing the pharmacokinetic assays of the kind that can be used in the present invention (in each case, the quencher used in the invention is not shown).

Unless indicated otherwise, all steps performed in the Experimental Part below were performed according to the manufacturer's instructions or otherwise using standard conditions generally known to the skilled person.

### Example 1: use of quenchers in a PK assay using the Mesoscale Discovery^{™} (MSD) platform.

This example illustrates the use of a quencher in pharmacokinetic assays used to determine total concentration of Nanobody-based (fusion) proteins in a human serum sample. In this example, the Mesoscale Discovery platform is used to determine the concentration of ALX-0171 (a trivalent Nanobody construct against human respiratory syncytial virus; see WO 2010/139808) in human serum samples spiked with different concentrations of ALX-0171. The assay set-up is essentially as schematically represented in Figure 1 (albeit that ALX-0171 is a trivalent Nanobody construct instead of the bivalent Nanobody construct shown for illustration purposes in Figure 1).

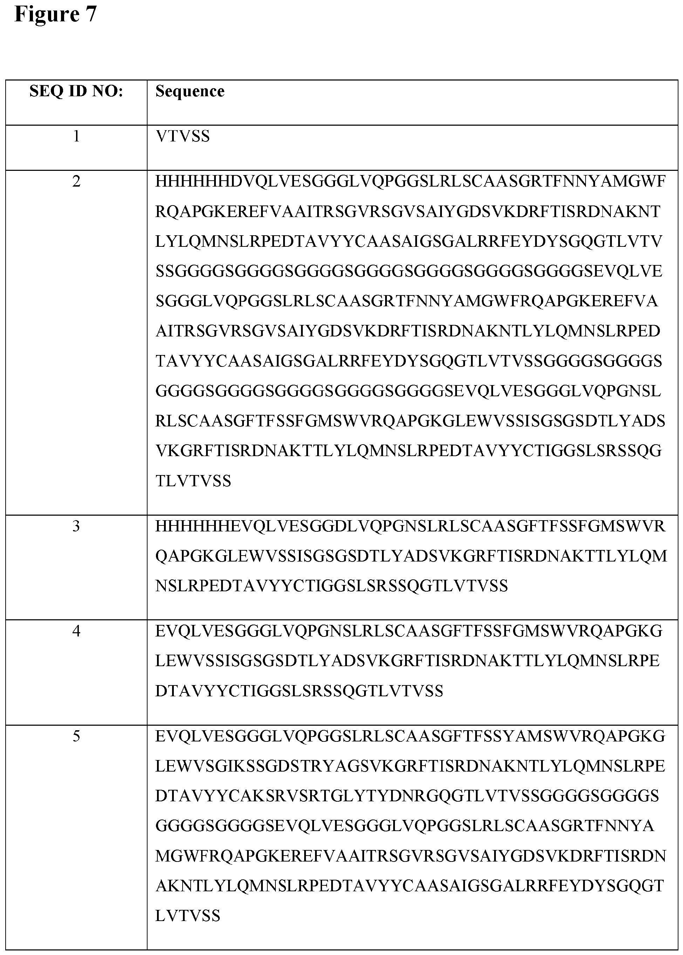

The quencher used was a trivalent Nanobody construct (with an N-terminal HIS-tag and a C-terminal end which was the sequence of SEQ ID NO: 1 (i.e. without any C-terminal extension)), the sequence of which is given in Figure 7 as SEQ ID No:2.

A biotinylated mouse monoclonal binding to and neutralizing ALX-0171 Nanobody was used as the capturing agent (at 5.0 microgram/ml in PBS/0.1% casein) and was immobilized on a Streptavidin-Gold multi-array 96-well plate. The human serum samples to be tested were diluted 50-fold (first 5-fold in PBS/0.1% casein, then 5-fold in 1000mM acetic acid. After incubation for 60 minutes at RT, dilute 2-fold in 1M Tris-buffer pH 9.5 containing 4,0 microgram quencher/ml). After overnight incubation at RT, samples are applied (in aliquots of 50 microliter) to the 96-well plates coated with the capturing agent under standard conditions that allow for ALX-0171 to be captured by the capturing agent.

After incubating for 1 hour and washing, the detection agent (a SULFO-tagged mouse monoclonal binding and neutralizing ALX-0171 Nanobody) was added and allowed to bind to the ALX-0171 captured by immobilized capturing agent. After washing, the amount of electroluminescent signal in each well of the plate was measured within 10 minutes after addition of MSD Read buffer using a Sector Imager 2400.

It was found that neither the presence of any pre-existing antibodies in the human serum samples used nor the presence or use of the quencher as part of the assay affected in any significant manner the determination of the concentration of ALX-0171 when ALX-0171 was spiked into the human serum samples at known concentrations between 987 pg/mL and 658537 pg/mL. These concentrations fall within a general concentration range that, inter alia, should be representative for the concentrations of Nanobody-based therapeutics which are expected to be encountered in serum samples obtained from human subjects during clinical trials involving the Nanobody-based therapeutic (e.g. for the purposes of determining the PK of the Nanobody-based therapeutic), optionally after suitable dilution.

### Example 2: use of quenchers in an ELISA-based PK assay.

This example illustrates the use of a quencher in ELISA-based pharmacokinetic assays used to determine total active concentration of Nanobody-based (fusion) proteins in a human serum sample.

In this example, the Nanobody is a bispecific construct directed against the IL-6 receptor and human serum albumin (see WO 2008/020079 and WO 2009/095489). As is well-known, the IL-6 receptor is known to occur both in a membrane-bound form as well as in a soluble form. The assay described in this example is capable of determining the total active concentration including "free" Nanobody (i.e. not bound to soluble IL-6 receptor that is present in the plasma sample used) and Nanobody that is bound to soluble IL-6 receptor that is present in the sample.

The assay set-up is essentially as schematically shown in Figure 4.

The quencher used was a monovalent Nanobody (with an N-terminal HIS-tag and a C-terminal end which was the sequence of SEQ ID NO:1 without any C-terminal extension), the sequence of which is given in Figure 7 as SEQ ID No:3.

A bivalent Nanobody recognizing the framework(s) of Nanobodies was used as the capturing agent (at 3.0 microgram/ml in BICA buffer) and was coated on a C96 Maxisorp plate. The human serum samples to be tested were diluted 200-fold (first 20-fold in PBS/0.1% casein, then 5-fold in 1600mM acetic acid. After incubation for 90 minutes at RT, dilute then 2-fold in 1M Tris-buffer pH 9.5 containing 0.5 microgram/ml human sIL-6 Receptor and 4,0 microgram quencher/ml).After 60 min incubation at RT, samples are applied (in aliquots of 100 microliter) to the 96-well plates coated with the capturing agent under standard conditions that allow for the anti-IL-6R Nanobody to be captured by the capturing agent.

After incubating for 1 hour and washing, a solution of soluble IL-6 receptor (0.25 microgram/ml) was added and incubated for 30 min at RT. After washing the plate, the detection agent (0.25 µg/ml of a mouse monoclonal recognizing a different epitope on IL-6R than the Nanobody allowing simultaneous binding of antibody and Nanobody) was added and allowed to bind to the IL-6R captured by the Nanobody, which by itself is bound by the immobilized capturing agent. After washing, 0.65 µg/ml of an HRP-labeled rabbit anti-mouse Ig was added and incubated for 30 min at RT. After washing TMB substrate was added and the OD signal in each well was measured at 450 nm using 620 nm as reference.

It was found that neither the presence of any pre-existing antibodies in the human serum samples used nor the presence or use of the quencher as part of the assay affected in any significant manner the determination of the concentration of the anti-IL-6R Nanobody when it was spiked into the human serum samples at known concentrations between 20.0 ng/mL and 1483.1 ng/mL. These concentrations fall within a general concentration range that, inter alia, should be representative for the concentrations of Nanobody-based therapeutics which are expected to be encountered in serum samples obtained from human subjects during clinical trials involving the Nanobody-based therapeutic (e.g. for the purposes of determining the PK of the Nanobody-based therapeutic), optionally after suitable dilution.

### Example 3: use of quenchers in a PK assay using the Mesoscale Discovery^{™} (MSD) platform.

This example illustrates the use of a quencher in pharmacokinetic assays used to determine total active concentration of Nanobody-based (fusion) proteins in a human serum sample. In this example, the Mesoscale Discovery platform is used to determine the concentration of ALX-0761 (a trivalent Nanobody construct against interleukin 17 A and F) in human serum samples spiked with different concentrations of ALX-0761.

The assay set-up is essentially as schematically shown in Figure 3. The quencher used was a monovalent Nanobody (which has a C-terminal end having the sequence of SEQ ID NO: 1 without any C-terminal extension), the sequence of which is given in Figure 7 as SEQ ID No:4.

A biotinylated mouse monoclonal recognizing the frameworks of Nanobodies was used as the capturing agent (at 1.0 microgram/ml in PBS/0.1% casein) and was immobilized on a Streptavidin-Gold multi-array 96-well plate. The human serum samples to be tested were diluted 100-fold (first 50-fold, then 2-fold, both in PBS/0.1% casein, containing 20.0 microgram quencher/mL and 50.0 microgram anti-IL-17 mAb/mL, which was added to prevent any free IL-17 to interfere with the assay). After overnight incubation at RT, samples are applied (in aliquots of 50 microliter) to the 96-well plates coated with the capturing agent under standard conditions that allow for ALX-0761 to be captured by the capturing agent.

After incubating for 1 hour and washing, a solution of soluble IL-17A (2.0 microgram/mL) was added and incubated for 1 hour at RT. After washing the plate, the detection agent (0.25 µg/ml of a SULFO-tagged mouse monoclonal binding the target IL-17A) was added and allowed to bind IL-17A captured by the Nanobody, which by itself is bound by the immobilized capturing agent. After washing, the amount of electroluminescent signal in each well of the plate was measured within 10 minutes after addition of MSD Read buffer using a Sector Imager 2400.

It was found that neither the presence of any pre-existing antibodies in the human serum samples used nor the presence or use of the quencher as part of the assay affected in any significant manner the determination of the concentration of ALX-0761 when ALX-0761 was spiked into the human serum samples at known concentrations between 99.7 ng/mL and 3280.9 ng/mL. These concentrations fall within a general concentration range that, inter alia, should be representative for the concentrations of Nanobody-based therapeutics which are expected to be encountered in serum samples obtained from human subjects during clinical trials involving the Nanobody-based therapeutic (e.g. for the purposes of determining the PK of the Nanobody-based therapeutic), optionally after suitable dilution.

### Example 4: use of quenchers in an ELISA-based PK assay.

This example illustrates the use of a quencher in ELISA-based pharmacokinetic assay used to determine concentration of Nanobody-based (fusion) proteins in a mouse plasma sample.

In this example, the Nanobody is directed against Her3. The assay described in this example is capable of determining the concentration ALX-0751 that is present in the sample.

The assay set-up is essentially as schematically shown in Figure 5.

The quencher used was a bispecific Nanobody construct (which has a C-terminal end having the sequence of SEQ ID NO:1 without any C-terminal extension), the sequence of which is given in Figure 7 as SEQ ID No:5.

In this ELISA, HER3-ECD (1.5 microgram/mL in 10:10 Trizma NaCl buffer) is coated on a C96 Maxisorp plate. The mouse plasma samples to be tested are diluted 20-fold in PBS/0.1% casein containing 200 microgram quencher per mL. After a 2 hour incubation at 37°C, samples are applied (in aliquots of 50 microliter) to the 96-well plates coated with the capturing agent (target) under standard conditions that allow for ALX-0751 to be captured by the capturing agent.

After incubating for 1 hour and washing, a detection reagent (1 microgram/mL biotinylated Nanobody recognizing the framework of the ALX-0751 Nanobody) was added and incubated for 1 hour at RT. After washing HRP labelled streptavidin is added and incubated for 30 min at RT. After washing TMB substrate was added and the OD signal in each well was measured at 450 nm using 620 nm as reference.

It was found that neither the presence of any pre-existing antibodies in the mouse plasma samples used nor the presence or use of the quencher as part of the assay affected in any significant manner the determination of the concentration of the ALX-0751 Nanobody when it was spiked into the mouse plasma samples at known concentrations between 24.9 ng/mL and 266.7 ng/mL. These concentrations fall within a general concentration range that, inter alia, should be representative for the concentrations of Nanobody-based therapeutics which are expected to be encountered in plasma samples obtained from mice during preclinical studies involving the Nanobody-based therapeutic (e.g. for the purposes of determining the PK of the Nanobody-based therapeutic), optionally after suitable dilution.

### Example 5: Protocol for determining affinity for 21-4.

Binding measurements were performed using a Biacore T100 using a CM5 sensor chip, with running buffer HBS-EP+, 25°C. 21-4 was captured via immobilized rabbit anti-mouse IgG, as it was found that directly immobilized mAb 21-4 surface could not efficiently be regenerated. The anti-mouse IgG used was a polyclonal rabbit anti-mouse IgG antibodies reacting with all IgG subclasses, IgA and IgM (GE Healthcare; Cat#BR-1008-38; Lot#10111487). Immobilisation of the anti-mouse IgG was performed using manual amine coupling using a 7 minute injection of EDC/NHS for activation and a 7 minute injection of 1M ethanolamine HCl pH 8.5 for deactivation (Biacore, amine coupling kit). Binding conditions are listed in Table I. Based on the immobilization level and MW of the proteins, the theoretical Rmax for mAb21-4 binding to the immobilized anti-mouse IgG was ~13000RU (when one mAb21-4 molecule is binding to one anti-mouse IgG molecule).

**Table I**

| **Flow cell (FC)** | **Protein** | **Conc. (µg/ml)** | **Contact time (s)** | **Flow rate (µl/min)** | **Immobilization buffer** | **Immobilization level (RU)** |
|---|---|---|---|---|---|---|
| FCl | Anti-mouse IgG | 30 | 420 | 5 | 10mM acetate pH5.0 | 17305 (after deactivation: 13440) |
| FC2 | Anti-mouse IgG | 30 | 420 | 5 | 10mM acetate pH5.0 | 17020 (after deactivation: 12820) |

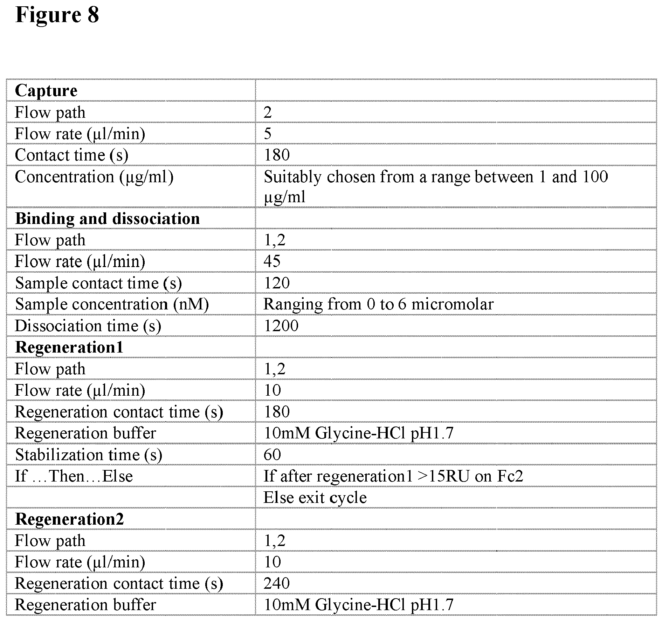

The conditions used for the binding experiment (Biacore T100) using 21-4 immobilized in the manner are given in Figure 8. The anti-mouse IgG surface could successfully be regenerated after capture of mAb 21-4 and injection of all samples (with a limited increase for baseline level after each regeneration).

The above protocol was used to generate the 21-4 affinity data for different quenchers as set out in Table II below.

**Table II**

| **Capture** | **Quencher** | **kₐ (1/Ms)** | **k_{d} (1/s)** | **K_{D} (M)** |
|---|---|---|---|---|
| 21-4 | SEQ ID NO:2 | 1.0E+04 | 3.0E-03 | **3.0E-07** |
| | SEQ ID NO: 3 | 6.7E+03 | 3.2E-03 | **4.8E-07** |
| | SEQ ID NO:4 | 4.5E+03 | 4.0E-03 | **8.9E-07** |
| | SEQ ID NO: 5 | 9.5E+04 | 8.2E-04 | **8.6E-09** |

## Claims

1. Method for determining the amount and/or concentration in a sample of at least one ISVD, or of a protein or polypeptide that comprises at least one ISVD, which method comprises the steps of;
a) contacting the sample with a capturing agent, such that the ISVD, protein or polypeptide is captured by said capturing agent;
b) contacting the ISVD, protein or polypeptide captured by the capturing agent with a detection agent, such that said detection agent binds to the ISVD, protein or polypeptide captured by the capturing agent;
c) generating a signal corresponding to the amount of detection agent bound to the ISVD, protein or polypeptide captured by the capturing agent,
in which said method is performed in the presence of a quencher, said quencher being a protein or polypeptide to which pre-existing antibodies can bind but to which neither the capturing agent nor the detection agent can bind.

2. Method according to claim 1, in which the protein or polypeptide used as the quencher is a protein or polypeptide that is bound by the monoclonal antibody 21-4 with an affinity better than 1 micromolar (µM), such as between 1000 and 1 nM.

3. Method according to claim 1 or 2, in which the protein or polypeptide used as the quencher is a protein or polypeptide is bound by the intended capturing agent and the intended detection agent with an affinity of less/worse than 3 micromolar, preferably less than 10 micromolar, more preferably less than 50 micromolar, such as worse than 100 micromolar.

4. Method according to any of claims 1 to 3, in which the quencher is a monovalent immunoglobulin single variable domain or a fusion protein or construct comprising not more than five, such as four, three, two or one immunoglobulin single variable domain(s).

5. Method according to claim 4, in which at least one of the immunoglobulin single variable domains present in the quencher has an exposed C-terminal end.

6. Method according to claim 5, in which the at least one of immunoglobulin single variable domain that has an exposed C-terminal end has the sequence VTVSS (SEQ ID NO:1) at its C-terminal end.

7. Method according to claim 4 or 5, in which at least the quencher has an immunoglobulin single variable domain at its C-terminal end.

8. Method according to claim 7, in which the immunoglobulin single variable domain at the C-terminal end of the quencher (and, by extension, the entire quencher) has the sequence VTVSS (SEQ ID NO: 1) at its C-terminal end.
